# EUROPÄISCHE PATENTSCHRIFT

(11) **EP 2 376 094 B1**
(45) Veröffentlichungstag und Bekanntmachung des Hinweises auf die Patenterteilung: **11.10.2017**
(21) Anmeldenummer: 10700330.3
(22) Anmeldetag: 14.01.2010
(51) Int. Cl.: A61K 36/33

(54) **VERFAHREN ZUR HERSTELLUNG EINES KAKTUSFRUCHTEXTRAKT**
PROCESS FOR THE MANUFACTURING OF A CACTUS FRUIT EXTRACT
PROCEDE POUR LA PREPARATION D'UN EXTRAIT DE FIGUE DE BARBARIE

(30) Priorität: 15.01.2009 AT 562009
(43) Veröffentlichungstag der Anmeldung: 19.10.2011
(73) Patentinhaber: Kaahee Research And Development GmbH, 1050 Wien (AT)
(72) Erfinder: RAUCHDOBLER, Julian, A-4060 Leonding (AT)
(74) Vertreter: Redl, Gerda
(86) Internationale Anmeldenummer: PCT/EP2010/050366
(87) Internationale Veröffentlichungsnummer: WO 2010/081839

(56) Entgegenhaltungen:
- EP-A1- 2 057 994
- WO-A1-03/037324
- WO-A1-2008/038849
- WIESE J ET AL: "EFFECT OF OPUNTIA FICUS INDICA ON SYMPTOMS OF THE ALCOHOL HANGOVER" ARCHIVES OF INTERNAL MEDICINE, AMERICAN MEDICAL ASSOCIATION, CHICAGO, IL, US LNKD- DOI:10.1001/ARCHINTE.164.12.1334, Bd. 164, 28. Juni 2004 (2004-06-28), Seiten 1334-1340, XP008043703 ISSN: 0003-9926 in der Anmeldung erwähnt

## Beschreibung

Die Erfindung betrifft ein Verfahren zur Herstellung eines Kaktusfruchtextraktes und dessen Verwendung zur Formulieren eines Getränkes.

Die Opuntien sind eine Pflanzengattung aus der Familie der Kakteengewächse (Cactaceae), die etwa 190 Arten umfasst. Die Nutzung einiger Opuntienarten als Nahrungsmittel ist bereits über mehrere hundert Jahre bekannt.
Extrakte aus Pflanzenteilen der Opuntia ficus indica ("OFI") werden zur Behandlung einer Reihe von Krankheitszuständen eingesetzt. So wird etwa dem Pflanzenextrakt eine pharmakologische Wirkung zur Behandlung von Nervenerkrankungen oder Stoffwechselstörungen nachgesagt.

WO03/037324A1 beschreibt einen Äthylacetat Extrakt von Opuntia ficus indica Frucht, Stamm oder getrockneter Frucht, zur Vorbeugung und Behandlung von Alzheimer, Schlaganfall, Parkinson, durch Ischämie verursachte Zell- und Gewebsschäden oder Herz-Kreislauferkrankungen wie z. B. auch Herzinfarkt. Dabei wird die anti-oxidative Wirkung des Opuntia ficus indica Extraktes zum Schutz von neuralen Schädigungen an Nervenzellen eingesetzt.

WO2008/038849A1 beschreibt ein Butanolextrakt oder Säurehydrolysat von Opuntia ficus indica welche als Tablette, Pille, Kapseln, Puder, Elixier, Lösung, Sirup oder Aerosol verabreicht werden kann und in Form einer pharmazeutischen Komposition zur Behandlung von Nervenerkrankungen, wie Erkrankungen der Hirnnerven, cerebrovaskulärer Erkrankungen, kardiovaskulärer Erkrankungen, Schlaganfall, Gehirnerschütterung, Alzheimer, Parkinson oder Herzinfarkt eingesetzt werden kann.

WO2005/041994A1 bzw. DE10350194A1 beschreibt die Verwendung von Pflanzenteilen von Opuntien und daraus hergestellte Extrakte, zur Behandlung von depressiven Verstimmungen und Erkrankungen oder von anderen affektiven Störungen, die durch Antidepressiva beeinflussbar sind wie z. B. Angst und Panikstörungen, Bipolaren Depressionen, Somatisierungsstörungen und des prämenstruellen Syndroms, sowie von Vorstufen derartiger Erkrankungen.

US2002/0102317A1 beschreibt die Gewinnung von biologischen Substanzen aus Opuntia ficus indica durch Extraktion der Haut der getrockneten Frucht mit einem organischen Lösungsmittel.

EP2057994A1 beschreibt ein Verfahren zur Herstellung eines Extraktes aus Feigenkakteen, durch Extrahieren von Pflanzenteilen.

Wiese et al. (Arch Intern Med 2004: 164, 1334-1340) beschreibt Kapseln, die einen Extrakt der Schale der Frucht von Opuntia ficus indica Frucht beinhalten und aufgrund dessen anti-inflammatorischer Wirkung zur Behandlung von Veisalgia eingesetzt werden. Die Wirksamkeit konnte in der Studie von Pitter et al. (BMJ 2005: 331,1515-1518) jedoch nicht bestätigt werden.

Im Allgemeinen werden in der Literatur bevorzugt Opuntia Blüten zur Herstellung von Extrakten mit pharmakologischer Wirkung eingesetzt. So wird etwa gemäß der WO2008/120206A1 ein wasserlöslicher alkoholischer Extrakt der Blüte von Opuntia ficus indica in Kapselform mit einer Tagesdosis von 0,003 bis 1g pro Person verabreicht, zur Inhibition von Alpha1-Adrenorezeptor.

Bekannte Extrakte von Opuntia ficus indica werden üblicherweise aufgrund der wasserunlöslichen Wirkstoffe und der Herstellung mit organischen Lösungsmitteln, wie Alkohol oder Butanol, als Trockenpräparate, wie z.B. Kapseln, oder hochkonzentrierte alkoholische Lösungen, wie z.B. Elixire, bereitgestellt. Diese Verabreichungsform ist wird jedoch oftmals von Patienten abgelehnt, insbesondere zur Behandlung von leichten Krankheitszuständen, wie Veisalgia.

Die Symptome von Veisalgia äußern sich im Unwohlsein und der Beeinträchtigung der körperlichen und geistigen Leistungsfähigkeit eines Menschen infolge einer leichten Alkoholintoxikation. Umgangssprachlich sind auch die Begriffe Kater oder Katzenjammer gebräuchlich. Die auslösende Alkoholmenge für die Symptome kann dabei von Mensch zu Mensch stark variieren. Die beschriebenen Symptome können die Leistungsfähigkeit von Stunden bis zu Tagen beeinträchtigen. Zum Krankheitsbild der Veisalgia zählen Kopfschmerzen, ein flaues Gefühl im Magen, ausgetrockneter Mund, Übelkeit bzw. Erbrechen und allgemeinem Unwohlsein. Häufig kommt durch eine Gastritis (Magenschleimhautreizung) Erbrechen hinzu, verbunden mit Appetitlosigkeit. Die geistigen und motorischen Fähigkeiten können eingeschränkt sein (z. B. Antriebslosigkeit, Schwindelgefühl, Konzentrationsschwierigkeiten, Gedächtnisausfall, leichtes Zittern). Teilweise treten depressive Verstimmungen bis hin zu Angstzuständen auf.
Die Aufgabe der vorliegenden Erfindung ist es, eine alternative Formulierung eines Extraktes aus Opuntia ficus indica zur Verfügung zu stellen, die es ermöglicht den Extrakt als Nahrungsergänzungsmittel oder auch als Genussmittel in einfacher Weise zu verabreichen, welcher für leichte Krankheitszustände wie beispielsweise Veisalgia, oder bereits zur Vorbeugung von Veisalgia eingenommen werden kann.

Diese Aufgabe wird erfindungsgemäß gelöst durch ein Verfahren zur Herstellung einer wässerigen Formulierung eines Kaktusfruchtextraktes gemäß Anspruch 1. Das unerwünschte Extraktionsmittel wird bevorzugterweise durch Überführen in einen glycolischen Extrakt entfernt.

Als Lösungsmittel wird ein C1-C4-Alkohol, insbesondere Ethanol oder Butanol, Ethylacetat oder Aceton eingesetzt. Zur Extraktion werden übliche Verfahren zur Pflanzenextraktion herangezogen, wobei hier von der Kaktusfrucht, bevorzugt in geschälter Form, aber auch als ganze Frucht mit Schale oder nur als Fruchtschale, ausgegangen wird. Das Ausgangsmaterial kann nur die Frucht, vorzugsweise gewaschen und zerkleinert, aber auch auch weiteres Pflanzenmaterial beinhalten, wie Blüten, Stamm, Blätter, Blattsprosse oder auch Samen. Die Frucht kann als frische Frucht oder getrocknet zerkleinert werden und wird dann in stufenweiser Form mit dem Extraktionsmittel behandelt. Beispielsweise wird die Frucht in einem 2- oder mehrstufigen Verfahren mit dem Lösungsmittel extrahiert, wobei mit einem Lösungsmittel/ Wassergemisch mit einer hohen Konzentration beginnend stufenweise reduziert wird, etwa im Bereich von 70 Vol%. Zur fraktionierten Extraktion mit Extraktionsmitteln verschiedenen Alkoholgehalts kann bei der ersten Fraktion die Flüssigkeit der Frucht als Teil des Extraktionsmittels dienen. Dann wird jeweils mit Alkohol-Wassergemischen weiterextrahiert.
Durch schrittweise Senkung des Alkoholgehalts werden auch hydrophilere Inhaltsstoffe gewonnen, sodass das Wirkstoffspektrum wesentlich breiter wird. Auf diese Weise werden neben den verschiedensten Betalainen zusätzlich Polyphenole, aber auch stickstoffhaltige Verbindungen wie Taurin und freie Aminosäuren mitextrahiert. Es konnte mit dieser stufenweisen Extraktion gezeigt werden, obzwar mit einem hohen Alkoholgehalt, z.B. mit über 50 Vol%, z.B. mehr als 60 Vol%, insbesondere etwa 70 Vol % Alkohol, beginnend, einen konzentrierten Extrakt mit definierten Wirkstoffen herzustellen, der trotzdem klar oder nur schwach opaleszierend mischbar ist.
Verschiedene Polysaccharide oder Hydrocolloide, z.B. Pektin, aber auch lipophile Bestandteile, z.B. Sterole oder fettlösliche Vitamine, die für wässerige Getränke eher ungeeignet sind und gegebenenfalls die Aufnahme der gewünschten Wirkstoffe verzögern können, werden durch die bevorzugte Alkoholextraktion nicht mitextrahiert.

Bevorzugterweise wird eine mehrstufige Extraktion in Form einer primären Mazeration mit etwa 70 Vol% Ethanol und nachfolgenden Perkolationen mit abgestuftem Alkoholgehalt bis etwa 45 Vol% vorgenommen, um die besten Ausbeuten mit dem interessantesten Wirkstoffspektrum zu erhalten.

Zusätzlich gelingt es durch den ersten Extraktionsschritt, der Mazeration bei einem Alkoholgehalt von mehr als 60 Vol%, z.B. bei etwa 70 Vol%, eine Entkeimung ohne Konservierungsmittel durchzuführen, die bei niedrigerem Alkoholgehalt nicht möglich ist. Dies ist insofern von Bedeutung, weil dieser Extrakt auch in nichtalkoholischen und nicht konservierten Getränken eingesetzt werden kann. Weiter konnten auch unerwünschte Begleitstoffe, wie Schleimstoffe, entfernt werden.

Als nächster Schritt wird der aus der alkoholischen stufenweisen Extraktion erhaltene Extrakt gegebenenfalls mit Glycerin versetzt, und der Alkohol beispielsweise unter Vakuum abdestilliert. Der Extrakt wird dann vorteilhafterweise filtriert.

Nach dem erfindungsgemäßen Verfahren ist es beispielsweise möglich, konzentrierte glycolische Extrakte aus frischen Opuntienfrüchten ohne Ethanolgehalt herzustellen, die sich in wässrigen Medien klar lösen.

Ein erschöpfendes Extraktionsverfahren kann von Vorteil sein, um die Inhaltsstoffe in konzentrierter Form zu erhalten.

Vorzugsweise wird ein Drogen bzw. Frischfrucht - Extraktionsverhältnis von mindestens 1:1 eingestellt, insbesondere in Verhältnis von 2:1 bis 5:1, vorzugsweise etwa 3:1. Das Extrakt enthält bevorzugterweise 10 bis 20% Feststoffe bzw. 20-50% Trockenstoffe (nicht verflüchtigbare Stoffe, bei 100-105°C gemäß Ph. Eur).

Nach der Extraktion wird das organische Lösungsmittel soweit abgetrennt, dass eine wässerige Lösung entsteht. Eine wässerige Lösung im Sinne der Erfindung wird als Formulierung verstanden, die im wesentlichen frei von den organischen Lösungsmitteln ist, die zur Extraktion eingesetzt wurden, insbesondere mit einem Gehalt an organischen Lösungsmittel von weniger als 5 Vol%, vorzugsweise, weniger als 3 Vol%, weniger als 2 Vol%, weniger als 1 Vol%, am meisten bevorzugt weniger als 0.5 Vol%. Ein Ethanolgehalt im Extrakt wird bevorzugterweise unter 1% gebracht, im Falle von Aceton oder Methanol sollte der Gehalt der organischen Extraktionsmittelbestandteile 0,05 Vol% nicht übersteigen. In der Regel wird diese Formulierung als alkoholfreies Getränk zur Verfügung gestellt. Weiter kann das Extrakt auch als Zusatz zu alkoholischen Getränken und als Konzentrat zum Einsatz kommen. Überraschenderweise hat sich gezeigt, dass sich die wesentlichen Inhaltsstoffe des Extraktes, wie Betalaine, z.B. Betanin, Phyllocactin, Indigoxanthin sowie Polyphenole, aber auch organische Säuren, Taurin, Prolin, Glutamin und andere freie Aminosäuren, in der wässerigen erfindungsgemäßen Formulierung wiederfinden, auch bei geringem oder ohne Alkoholgehalt. Diese sind durch Extraktion der OFI Frucht nur mit Wasser nicht erhältlich.

Das Lösungsmittel wird vorzugsweise durch Phasentrennung bzw. Evaporation abgetrennt, in besonderen Fällen auch durch Sprühtrocknen. Nach Einengung des Extraktes wird dieser je nach Verwendungszweck formuliert und gegebenenfalls haltbar gemacht.

Ein trockener Extrakt bzw. gefriergetrockneter Extrakt, aber auch die getrocknete erfindungsgemäße Formulierung, kann gegebenenfalls über einen längeren Zeitraum vor der Aufnahme mit Wasser bzw. mit einer wässerigen Zubereitung gelagert werden. Die Trockenformulierung ist vorzugsweise auch als Handelsprodukt zur späteren Abfüllung geeignet. Es wird eine geeignete Formulierung zur Verfügung gestellt, die dem Verwendungszweck des Getränkes entspricht. So kann zur Vorbeugung von Veisalgia ein hochkonzentriertes Elixier bereitgestellt werden, zur Behandlung bereits auftretender Krankheitsbilder jedoch ein verdünntes Getränk, um den erhöhten Wasserbedarf abzudecken. Ebenso sind oral verabreichbare pharmazeutische Fertigformulierungen, z.B. eine Lösung, Sirup, aber auch Trockenpulver, Granulat oder Brause zur Aufnahme mit Wasser möglich.
Ein Extraktgehalt von mindestens 2,5 Gew% zur Aufnahme einer genügenden Dosis mit pharmakologischer Wirkung wird bevorzugt. Die bevorzugterweise eingesetzte Konzentration liegt bei mindestens 3 Gew%, vorzugsweise bei mindestens 5 Gew%, weiter bevorzugt mindestens 7 Gew%, 9 Gew% oder 10 Gew%, bis zu 20 Gew%. Für spezielle Formulierungen kann eine Konzentration bis zu 30 Gew% gewählt werden. Besonders bevorzugte Formulierungen weisen eine Konzentration von 3,2 Gew% auf, für eine bevorzugte tägliche Dosis von etwa 5-30 g, insbesondere bevorzugt 3-10 g. Konzentrate werden auch mit einer Konzentration von etwa 14-16 Gew% angeboten, um eine bevorzugte tägliche Dosis von bis zu 50 g zur Verfügung zu stellen. Dabei ist darauf zu achten, dass die Formulierung die Wirkstoffe in gelöster Form enthält, um Schwebstoffe zu vermeiden, die zu einem Absetzen des Extraktes im Gebinde führt. Es kann jedoch auch von Vorteil sein, wenn ein konzentriertes Getränk zur Verfügung gestellt wird, welches mit einem alkoholischen Getränk gemischt wird, um eine alkoholische Lösung der Inhaltsstoffe zu erhalten. Die erfindungsgemäße Formulierung kann auch als verdünntes "near water" Getränk zur Verfügung gestellt, vorzugsweise als klare Lösung.
Bevorzugterweise wird als Gebinde ein luftdichter Behälter aus Glas, PET oder Metall, z.B. Aluminium verwendet.

Weitere Inhaltsstoffe der Rezeptur sind vorzugsweise ausgewählt aus der Gruppe von Taurin, Anthocyane, Acaibeerenextrakt, Guarana, Grünteeextrakt, Koffein, Fructose, Saccharose, Ingwerbestandteile, Betanin, Vitamine, Vitamin B1 und/oder B12, Pantothensäure, insbesondere das Kalziumsalz, Niacin, Folsäure, Biotin, Pyridoxin und Vitamin C, Mineralstoffe, wie Kalzium oder Magnesium, Selen, Zink, Gluconat, Coenzym Q10, L-Carnithin, Liponsäure und Kohlensäure, sowie weitere Inhaltsstoffe, die in einer oral zu verabreichenden Formulierung bzw. in einem Getränk oder als Nahrungsergänzungsmittel üblicherweise zur Konsistenz, Aroma oder zur Geschmacksverbesserung, wie Zitronensäure, Ginkgo biloba - Extrakt oder natürliche Aromen, wie z.B. Kaktusfeige-Aroma, beitragen. Als Träger wird bevorzugt Maltodextrin verwendet.

Nach Möglichkeit wird der Zusatz von chemischen Konservierungsmitteln, wie Benzoate oder Sorbate vermieden.

Beispielsweise werden folgende Zusammensetzungen vorgeschlagen:

**Prophylaxe : "Up-Front" - Getränk**

| Extrakt Kaktusfeigen | ≥ 10 g/100 ml | |
|---|---|---|
| Taurin (Leberentgiftend) | 200 mg/100 ml | Wasserlöslich geruchgeschmacklos |
| Anthocyane/Acaibeerenextrakt (farbgebend, anti-oxidativ) | 100-200 mg/100 ml | Wasserlöslich geruchgeschmacklos (leicht säuerlich |
| Vitamine Niacin, Pyridoxin, Pantothensäure | 15 % RDA/100 ml | Wasserlöslich geruchgeschmacklos |
| Mineralstoff (Kalzium) | 15 % RDA/100 ml | Wasserlöslich geruchgeschmacklos |
| Coenzym Q10 ("energieliefernd) | 10 mg/100 ml | Wasserlöslich geruchgeschmacklos |

**Therapie: "Morning-After" - Getränk**

| Extrakt Kaktusfeigen | ≥ 10 g/100 ml | |
|---|---|---|
| Guarana oder Grünteeextrakt (Koffein natürlich) | 15 mg Koffein/ 100 ml | Wasserlöslich geruchlos, leicht bitter aber gut kaschierbar |
| Vitamine Thiamin, Vitamin C, Vitamin B12, Biotin, Folsäure | 50 % RDA/100 ml | Wasserlöslich geruchgeschmacklos |
| Mineralstoffmischung Zink, Selen, Magnesium | 50 % RDA/100 ml | Wasserlöslich geruchgeschmacklos |
| Getrocknete Ingwerwurzel (anti-Übelkeit) | 100 mg/100 ml | |
| Liponsäure (Energieliefernd) | 20 mg/100 ml | Wasserlöslich geruchgeschmacklos |

**Universell einsetzbares Produkt gemäß der "Ein-Produktstrategie"**

| Zutat | Menge/Einheit | |
|---|---|---|
| Extrakt Opuntia | > 10g/100ml | |
| Guarana (natürliches Koffein) | 50-100 mg Koffein | wasserlöslich, leicht gefärbt, leicht bitter |
| Zitronensäure | 0.5-1.0 g | wasserlöslich, sauer |
| Ginkgo biloba Extrakt oder natürliche Aromen | 0.1-0.5 g | wasserlöslich, leicht gefärbt, geschmacksneutral |
| Taurin | 0.2 g | wasserlöslich, ungefärbt, geschmacksneutral |
| Kalzium (org. Salz, Gluconat) | 0.2 g | wasserlöslich, ungefärbt, geschmacksneutral |
| Magensium (org Salz, Gluconat) | 0.18 g | wasserlöslich, ungefärbt, geschmacksneutral |
| Pantothenic Acid (Ca-Salz) | 1.8 mg | wasserlöslich, ungefärbt, geschmacksneutral |
| Niacin | 5.4 mg | wasserlöslich, ungefärbt, geschmacksneutral |
| Pyridoxin (HCl) | 0.6 mg | wasserlöslich, ungefärbt, geschmacksneutral |
| Vitamin C | 36 mg | wasserlöslich, ungefärbt, leicht säuerlich |

Die Formulierung wird vorzugsweise als haltbares Verkaufprodukt, mit einer Haltbarkeit von mindestens 6 Monaten, bevorzugterweise mindestens 12 Monaten bereitgestellt. Daher wird ein Verfahrensschritt zur Sterilisierung bzw. Konservierung vorgeschlagen, der vorzugsweise eine physikalische Behandlung umfasst. Insbesondere wird eine Wärmebehandlung bzw. Behandlung mit Säure, etwa bei einem pH im Bereich von 3.5 und 5.0, vorzugsweise pH 4, vorgeschlagen. Die bevorzugte Wärmebehandlung ist eine kurzzeitige Erhitzung auf Temperaturen bis zu 80°C, etwa im kontinuierlichen Verfahren eine Behandlung bei 72°C für die Dauer von 1-2 Minuten, oder bei geringeren Temperaturen für eine längere Zeit, etwa bei 60°C +/- 2°C für eine Dauer von 30 Minuten, vorzugsweise im Batch-Verfahren.
Eine schonende Behandlung zur Konservierung ist jedenfalls vorzuziehen, um die Inhaltsstoffe, wie Betalaine, Polyphenole oder stickstoffhaltigen Verbindungen, wie Taurine und freie Aminosäuren, nur unwesentlich herabzusetzen.

Die Formulierung wird zur Behandlung von Veisalgia zur Verfügung gestellt, insbesondere zur Prophylaxe und Therapie der Veisalgia, deren Symptome und damit assoziierten Krankheitszuständen. Die bevorzugte Dosis ist etwa zur Vorbeugung mindestens 100mol, bevorzugterweise mindestens 200ml des Getränkes, etwa bis zu 10 Stunden vor Alkoholkonsum, aber auch unmittelbar vor dem Trinken von Alkohol bzw. gemeinsam mit dem alkoholischen Getränk eingenommen. Zur Therapie ist bevorzugterweise innerhalb von 24 Stunden nach Alkoholgenuss, etwa innerhalb von 12 Stunden, eine Dosis von mindestens 200ml, vorzugsweise mindestens 300ml, aber auch mindestens 400ml oder mehr des Getränkes vorgesehen. Die maximale Tagesdosis wird etwa mit 2000ml geschätzt, um ein Unwohlsein aufgrund der Inhaltsstoffe zu vermeiden. Die Erfindung wird durch das nachfolgende Beispiel weiter beschrieben.

### Beispiel : Bereitung eines OFI Extraktes

Zur Herstellung des Extraktes wurden ganze, ungeschälte, jedoch von den Stacheln befreite, frische Opuntia Frucht verwendet. In der Schale ist die Hauptmenge der pharmakologisch wertvollen Inhaltsstoffe, wie Betalaine vorhanden. Die Frucht wird vor Verarbeitung gewaschen, wieder getrocknet und dann in einem Cutter zerkleinert.

Als Extraktionsverfahren kommt die fraktionierte Perkolation mit gradueller Verringerung des Alkoholanteils zur Anwendung. Da die Opuntienfrucht ca. 85 % Flüssigkeit enthält, muss, um einen definierten Extrakt zu bekommen, zuerst eine Trockenverlustbestimmung durchgeführt, um die zuzusetzende Menge von Ethylalkohol zu berechnen. Die Extraktionsflüssigkeit setzt sich bei der ersten Fraktion aus dem Flüssigkeitsanteil der Frucht und dem zugesetzten Alkohol zusammen. Es wird eine Teilmenge des Alkohols zugesetzt, um einen Fermentationsprozess zu vermeiden. Nachdem die notwendige Zusatzmenge des Alkohols bestimmt und berechnet ist, wird sofort die restliche Alkoholmenge zugesetzt und kurz gerührt. Bei der ersten Ansatzfraktion wird die Alkoholmenge auf etwa 70 Vol % (63 Gew %) festgelegt. Bei den nachfolgenden Fraktionen wird das Extraktionsgemisch schrittweise bis auf 45 Vol. % Alkoholgesenkt, wobei Alkohol mit Wasser vorher gemischt wird.

Zum Einengen des Extraktes werden die gesammelten Extraktfraktionen im Rotationsverdampfer unter Vakuum unter Zusatz von Ascorbinsäure eingeengt, bis die Extraktmenge einem Frischpflanzen/Extraktverhältnis von ca. 3: 1 entspricht und über 20 % Trockenrückstand enthält. Je nach Anwendungszweck wird dieser Extrakt mit einer Teilmenge Glycerin, Ethanol, Glucosesirup etc. aufgenommen und der Analytik zugeführt.

## Patentansprüche

1. Verfahren zur Herstellung einer wässerigen Formulierung eines Kaktusfruchtextraktes, enthaltend die folgenden Schritte:
a) zur Verfügung Stellen von Opuntia ficus indica Frucht,
b) stufenweises Extrahieren der Frucht mit einem Extraktionsmittel enthaltend einen C1-C4 Alkohol als organisches Lösungsmittel in einer Konzentration von mehr als 50 vol%, in einem 2- oder mehrstufigen Verfahren, wobei stufenweise die Lösungsmittelkonzentration verändert wird, und die Konzentration weiter auf eine Konzentration von bis zu 45 vol% reduziert wird,
c) Abtrennen des Lösungsmittels, und
d) Formulieren des Extraktes zu einer wässrigen Lösung, mit einem Gehalt an organischem Lösungsmittel von weniger als 5 Vol%, mit einer Konzentration von mindestens 2,5 Gew% Extrakt.

2. Verfahren nach Anspruch 1, **dadurch gekennzeichnet, dass** der Extrakt zu einer wässrigen Lösung mit einer Konzentration von 2,5 - 20 Gew% Extrakt formuliert wird.

3. Verfahren nach Anspruch 2, **dadurch gekennzeichnet, dass** der Extrakt zu einer wässrigen Lösung mit einer Konzentration von mindestens 5 Gew% Extrakt formuliert wird.

4. Verfahren nach einem der Ansprüche 1 bis 3, **dadurch gekennzeichnet, dass** das Lösungsmittel Alkohol oder Ethylacetat ist.

5. Verfahren nach einem der Ansprüche 1 bis 4, **dadurch gekennzeichnet, dass** die in Anspruch 1, Punkt d, erhaltene wässrige Lösung weiter durch eine physikalische Behandlung haltbar gemacht wird.

6. Verfahren nach Anspruch 5, **dadurch gekennzeichnet, dass** die physikalische Behandlung eine Säurebehandlung und/oder Pasteurisierung umfasst.

## Claims

1. A method for preparing an aqueous formulation of a cactus fruit extract, consisting of the following steps:
a) supplying Opuntia ficus indica fruit,
b) incrementally extracting the fruit with an extraction agent containing a C1-C4 alcohol as organic solvent in a concentration of more than 50 vol%, in a procedure involving 2 or more stages, wherein the concentration of the solvent is altered incrementally and the concentration is reduced further to a concentration as low as 45 vol%,
c) separating the solvent, and
d) formulatiing the extract to produce an aqueous solution with a content of organic solvent less than 5 vol%, with a concentration of at least 2.5 wt% extract.

2. The method according to claim 1, **characterised in that** the extract is formulated to produce an aqueous solution with concentration from 2.5 - 20 wt% extract.

3. The method according claim 2, **characterised in that** the extract is formulated to produce an aqueous solution with a concentration of at least 5 wt% extract.

4. The method according to any one of claims 1 to 3, **characterised in that** the solvent is alcohol or ethyl acetate.

5. The method according to any one of claims 1 to 4, **characterised in that** the aqueous solution obtained as described in claim 1, step d is further rendered stable by a physical treatment.

6. The method according to claim 5, **characterised in that** the physical treatment comprises an acid treatment and/or pasteurisation.

## Revendications

1. Procédé de préparation d'une formulation aqueuse d'un extrait de fruit de cactus, comprenant les étapes suivantes :
a) prévision du fruit opuntia ficus indica
b) extraction progressive du fruit à l'aide d'un moyen d'extraction contenant un alcool C1-C4 en tant que solvant organique à une concentration de plus de 50 % en volume, dans un procédé à 2 niveaux ou plus, la concentration en solvant étant modifiée progressivement et la concentration continuant à être réduite à une concentration allant jusqu'à 45 % en volume,
c) isolation du solvant, et
d) formulation de l'extrait en une solution aqueuse ayant une teneur en solvant organique de moins de 5 % en volume à une concentration d'au moins 2,5 % en poids.

2. Procédé selon la revendication 1, **caractérisé en ce que** l'extrait est formulé en une solution aqueuse ayant une concentration de 2,5 à 20 % en poids.

3. Procédé selon la revendication 2, **caractérisé en ce que** l'extrait est formulé en une solution aqueuse ayant une concentration d'au moins 5 % en poids.

4. Procédé selon une des revendications 1 à 3, **caractérisé en ce que** le solvant est de l'alcool ou de l'éthyle acétate.

5. Procédé selon une des revendications 1 à 4, **caractérisé en ce que** la solution aqueuse obtenue dans le revendication, point d, est en outre rendue apte à la conservation par traitement physique

6. Procédé selon la revendication 5, **caractérisé en ce que** le traitement physique comprend un traitement à l'acide et/ou une pasteurisation.
